# EUROPEAN PATENT APPLICATION

(11) **EP 4 759 919 A1**
(43) Date of publication of application: **17.06.2026**
(21) Application number: 24383362.1
(22) Date of filing: 12.12.2024
(51) Int. Cl.: C12N 1/14, C12N 9/00, C12N 13/00, C12P 1/00, C12P 7/00, C12N 15/01, C12R 1/885

(54) **FUNGAL ENZYMATIC COMPOSITION FOR DEGRADING PLASTIC PRODUCTS**

(71) Applicant: Entzimatiko, S.L., 20870 Elgoibar, Gipuzkoa (ES)
(72) Inventor: REYES, Mario, Elgoibar, GIPUZKOA (ES)
(74) Representative: Balder IP Law, S.L.

(57) **Abstract**

The invention relates to a fungal enzymatic composition for degrading plastic products and plastic polymers obtained from the simultaneous cultivation of at least three different species of Trichoderma fungi. Moreover, the present invention also relates to a method for the depolymerization and degradation of plastic-containing waste by the fungal enzymatic composition of the invention.

## Description

### TECHNICAL FIELD

The invention disclosed herein relates generally to the field of biodegradation and bioremediation. More specifically, the present invention relates to plastics depolymerization, biofragmentación and biodegradation. The invention relates to a method for the depolymerization and degradation of plastic-containing waste by a composition comprising encapsulated lytic enzymes obtained from the simultaneous cultivation of at least three different species of Trichoderma fungi.

### STATE OF THE ART

Plastic is found in several areas of industry, with diverse applications, such as food packaging, pharmaceuticals, toys, general household products, construction, decoration and countless other industries. Each plastic material has a different chemical composition, some of which are reusable, and others have a complex recycling process.

Plastic polymers are classified into two large distinct groups according to processing and thermal behavior: thermoplastics and thermosets. The former are moldable, as they soften when heated; and the latter are not easily moldable by heating. Among the plastics that are produced and used for various commercial purposes, there are polyethylene (PE)-based products, polypropylene (PP) products, polyvinyl chloride (PVC) products, polyurethane (PU) products, polystyrene (PS, styrofoam) products, polyvinyl chloride (PVC) products, polytetrafluoroethylene (Teflon), polyethylene terephthalate (PET or PETE or polyester) products, among others.

In 2018, the United Nations (UN) started a global awareness movement about the disposal of plastic objects in the environment and its effects. One of the main aggravating factors of pollution caused by plastic materials is due to the high half-life of this material, which sometimes takes more than 200 years to decompose in nature, which causes a strong environmental impact. Around 91 % of the plastic used worldwide is not recycled. According to the ASTM Standard D5033, plastic recycling can be categorized as primary, secondary, tertiary and quaternary (Standard Guide to Development of ASTM Standards Relating to Recycling and Use of Recycled Plastics. American Society for Testing and Materials (ASTM) International; West Conshohocken, PA, USA: 2000).

Each category involves distinct processes and objectives. Primary recycling, also known as closed-loop recycling, entails mechanically reprocessing plastic scrap to produce a product with properties equal to those of the original material. Secondary recycling, referred to as downgrading recycling, involves mechanically reprocessing plastic scrap to produce a product with altered properties. Tertiary recycling focuses on the recovery of chemical constituents from plastic scrap, while quaternary recycling harnesses the energy content of scrap plastic to generate steam and electricity. Mechanical recycling is used in both primary and secondary recycling. While most thermoplastics, including PET, PE, and PP, have high potential for mechanical recycling, thermosets such as unsaturated polyester and epoxy resin cannot be mechanically recycled due to their molecular structure. The varying processing requirements and molecular incompatibility of different plastic-types present challenges in the production of recycled plastic from plastic waste. The mechanical recycling process comprises several key stages, including collection, sorting, cleaning, size reduction, and compatibilization or separation.

Generally, the main recycling methods require high-temperature and high-pressure conditions (chemical methods) or consume a large amount of energy and/or generate a large amount of toxic and harmful substances to the environment, which also results in secondary pollution (biological).

To minimize these effects, based on an environmentally sustainable approach to the recycling of plastic products, biodegradation methods have been intensively applied and studied.

Recent research on biodegradation has addressed the ability of microorganisms to degrade plastic. With the current knowledge on plastic degradation through microbial means, it is known that enzymes secreted or obtained from microorganisms act mainly on PET and PU polymers (Wei R; ZimmermannW., Microb Biotechnol. 2017 Nov;10(6):1308-1322; Danso D., et al. Appl Environ Microbiol. 2019 Sep 17;85(19):e01095-19). Generally, microbial degradation of polymers is a slow process.

However, the best enzymes and microorganisms with activity in PU and PET still show moderate catalysis rates, therefore microbial degradation of polymers is still a slow process. This high resistance derives mainly from the fiber's high molecular weight, strong C-C interactions and the extremely hydrophobic surface, which is very difficult for enzymes to attack. Furthermore, polymers have different molecular orderings (e.g. amorphous and crystalline forms), resulting in different levels of degradability.

The first evidence of biodegradation of aromatic polyesters was reported in a study published in 2005, in which the microorganism *Thermobifida fusca* was used to degrade PET (MüllerJR., et al. Enzymatic degradation of Poly(ethylene terephthalate): rapid hydrolyse using a hydrolase form T. fusca. Macromolecular Rapid Communication 2005, 26, 1400-1405). From then on, other microorganisms were tested in the degradation of this substrate.

Other approaches to biodegradation consist of the use of enzymes isolated from microorganisms. Currently, there are many known functional and verified enzymes, such as PETases, proteases, lipases, carboxylesterases, cutinases and esterases, most of which are biochemically broken down according to the synthetic polymer substrates or oligomers/monomers to which they bind. Cutinase enzymes represent the largest dominant fraction within the studies performed. For example, enzymes have been reported Thermobifida alba Cut1 (ADV92525), Thermobifida fusca Cut2 (CBY05530), Thermobifida fusca Cut1 (AET05798), Thermobifida halotolerans Serine hydrolase (AFA45122), Saccharomonospora viridis Cutinase (BAO42836), Thermomonospora curvata triacilglicerol lipase (WP 012851645), LCC (AEV21261), Ideonella sakaiensis PETase (GAP38373), Polyangium brachysporum triacilglicerol lipase (WP047194864), Vibrio gazogenes lipase (WP021018894), LiplAF5-2 (ACC95208), Oleispira antarctica LipA (CCk74972), Ideonella sakaiensis MHETase (WP 082368715), Humicola insolens cutinase (4OYY) and Fusarium oxysporum cutinase (5AJH) degrade polyethylene substrates. For degradation of the polyamide (PA) substrate, enzymes have been applied Arthrobacter sp NylE (WP079941038), Arthrobacter sp NylA (BAA05090), Arthrobacter sp NylB (CAA24927) and Arthrobacter sp NylC (YP001965068). The enzymes Pseudomonas oleovorans AHs (CAB54050), Pseudomonas fluorescens StyB (CAB06823), Pseudomonas fluorescens StyA (CAB06823) and Pseudomonas fluorescens StyC (CAB06825) degraded polystyrene (PS), while the degradation of polyurethane (PU) can occur with Pseudomonas fluorescens PueA (AAC23718), Pseudomonas fluorescens PueB (AAY92474) and Pseudomonas fluorescens PulA (AAF66684) (Danso D. et al ppl Environ Microbiol. 2019 Sep 17;85(19):e01095-19).

Nonetheless, the use of fungal enzymes in plastic biodegradation is not well known, in contrast to the use of fungal enzymes obtained from fungi of the genus Trichoderma for the degradation of biomass or biological materials, such as cellulosic and lignocellulosic materials. In this sense, PCT application published as WO 2021205160 describes KatG enzymes, enzyme compositions, and their uses in the enzymatic degradation of plastics, enzymes that can be produced in host microorganisms that can include Trichoderma spp. However, KatG enzymes are different from enzymes produced naturally by Trichoderma.

Therefore, there is a demand for further improved fungal enzymes capable of degrading plastic polymers for use in bioremediation for the degradation of plastic waste.

### DESCRIPTION OF THE INVENTION

In a first aspect, the present disclosure provides a composition for degrading plastic products, hereinafter the composition of the invention, comprising:
a) an enzymatic extract obtained from a culture comprising at least three live *Trichoderma* species selected from the list consisting of *Trichoderma harzianum, Trichoderma viridae, Trichoderma longibratum koningii* and any variant thereof, cultivated all together, obtained in laboratory and/or at industrial scale, on micronised oat as solid means of cultivation, identified as T 22, Tr 115, Tr 116, KRL -AG 2 (Rifai), including holoforms as *Hypocrea* and *Podostroma,* wherein *T. longibratum* stump was transformed by means of the exposition to radiation before being cultured with the other *Trichoderma* species, and
b) at least a phosphatidine as a surfactant.
wherein the enzymatic extract and the surfactant are encapsulated within the composition.

The inventors of the present invention found that the composition of the invention, wherein the enzymatic extract is encapsulated therein, has a surprising and effective plastic degradation activity, demonstrating their use in the degradation of plastics. Equally surprising, the inventors of the present invention have demonstrated that the composition of the present invention exhibit plastic-degrading activity without the need for the concomitant use of canonical enzymes such as PETases, proteases, lipases, carboxylesterases, cutinases and esterases.

In a second aspect, the present disclosure relates to the use of the composition of the invention for degrading plastic products.

In a third aspect, the present disclosure relates to a method of degrading plastic products, hereinafter the method of degrading plastic product of the present invention, wherein the method comprising a depolymerization step, performed at room temperature, wherein the plastic product is contacted with the composition of the invention; and wherein the depolymerization step is conducted in an aqueous liquid medium. The method of the present invention occurs at room temperature, being advantageous in relation to the other methods of enzymatic degradation of plastic described in the state of the art.

### BRIEF DESCRIPTION OF THE DRAWINGS

To complete the description and in order to provide for a better understanding of the invention, a set of drawings is provided. Said drawings form an integral part of the description and illustrate aspects and embodiments disclosed herein, which should not be interpreted as restricting the scope of the invention, but just as an example of how the invention can be carried out. The drawings comprise the following figures:
**Figure 1** shows a photograph of an electrophoretic SDS-PAGE gel of the enzyme extract of the invention showing the presence of seven different proteins (numbers 1 to 7 shown on the right side of the figure) based on the molecular weight (kDa) of the control sample.
**Figure 2A** shows a scanning electron microscopy (SEM) image (500x) obtained from individual samples of the PET fragments used as a control (not treated with the composition of the invention).
**Figures 2B and 2C** show SEM images (500x and 1500x, respectively) obtained from individual samples of the PET fragments treated with the composition of the invention for 40 min at room temperature.
**Figures 2D and 2E** **show** SEM images (500x and 1500x, respectively) obtained from individual samples of the PET fragments treated with the composition of the invention for 60 min at room temperature.
**Figures 3A and 3B** show SEM images (100x and 1000x, respectively obtained from individual samples of the PET fragments used as a control (not treated with the composition of the invention).
**Figures 3C and 3D** show SEM images (100x and 1000x, respectively) obtained from individual samples of the PET fragments treated with the composition of the invention for 24 hours at room temperature
**Figures 4A and 4B** show SEM images (100x and 1000x, respectively obtained from individual samples of the PET fragments used as a control (not treated with the composition of the invention).
**Figures 4C and 4D** show SEM images (100x and 1000x, respectively) obtained from individual samples of the PET fragments treated with the composition of the invention for 24 hours at room temperature

### DETAILED DESCRIPTION OF THE INVENTION.

The following definitions and methods are provided to better define the present invention and to guide those of ordinary skill in the art in the practice of the present invention. Unless otherwise noted, terms are to be understood according to conventional usage by those of ordinary skill in the relevant art. Each aspect so defined may be combined with any other aspect or aspects unless clearly indicated to the contrary. In particular, any feature indicated as being preferred or advantageous may be combined with any other feature or features indicated as being preferred or advantageous.

As previously states, the present invention relates to a composition for degrading plastic products comprising:
a) an enzymatic extract obtained from a culture comprising at least three live *Trichoderma* species selected from the list consisting of *Trichoderma harzianum, Trichoderma viridae, Trichoderma longibratum,* and any variants thereof, identified as T 22, Tr 115, Tr 116, KRL -AG 2 (Rifai) and cultivated all together, obtained in laboratory and/or at industrial scale, on micronised oat as solid means of cultivation, , including holoforms as *Hypocrea* and *Podostroma,* wherein *T. longibratum* stump was transformed by means of the exposition to radiation before being cultured with the other *Trichoderma* species, and
b) at least a phosphatidine as a surfactant.

In the context of the invention, a "plastic product" refers to any item made from at least one polymer, monomer(s), hydrocarbons; such as plastic sheet, film, tube, rod, profile, shape, massive block, fiber, etc. Preferably, the plastic article is a manufactured product, such as a rigid or flexible packaging, agricultural films, bags and sacks, disposable items or the like. Preferably, the plastic article comprises a mix of semicrystalline and/or amorphous polymers, or semi- crystalline polymers and additives. The plastic articles may contain additional substances or additives, such as plasticizers, mineral or organic fillers. According to the invention, the plastic article may be selected from a plastic film or a rigid plastic article.

According to the invention, the term "plastic film" refers to a flexible sheet of plastic (i.e., capable of being flexed without breaking). Examples of plastic films include agricultural films, plastic bags or sacks, films for flexible packaging, food films, mailing films, liner films, multipack films, industrial films, personal care films, nets, etc.

According to the invention, the term "rigid plastic article" refers to a plastic article which is not a film. These articles are preferably produced by calendering, injection-molding, thermoforming, blow molding, or even by rotomolding and 3D printing. Examples of rigid plastic articles are thin wall packaging such as food and beverage packaging, boxes, trays, containers, food service ware, electronics casings, cosmetic cases, outdoor gardening items such as pots, rigid packaging, containers, cards, cotton swabs, irrigation pipes, etc.

A "polymer" refers to a chemical compound or mixture of compounds whose structure is constituted of multiple repeating units linked by covalent chemical bonds. Within the context of the invention, the term "polymer" includes natural or synthetic polymers, comprising a single type of repeating unit (i.e., homopolymers) or different types of repeating units (i.e., block copolymers and random copolymers). As an example, synthetic polymers include polymers derived from petroleum oil or biobased polymers, such as polyolefins, aliphatic or aromatic polyesters, polyamides, polyurethanes and polyvinyl chloride. Natural polymers include lignin and polysaccharides, such as cellulose, hemi-cellulose, starch and derivatives thereof that may or may not be plasticized. According to the invention, "oligomers" refer to molecules containing from 2 to about 20 monomer units.

Within the context of the invention, the term "polyester" can be a homopolymer or copolymer. As an example, polylactic acid is an aliphatic homopolymer composed of one monomer, lactic acid; and polyethylene terephthalate is an aliphatic-aromatic copolymer composed of two monomers, terephthalic acid and ethylene glycol. Such polyesters may be native or chemically modified. In the present description, "polyesters" encompass polyethylene terephthalate (PET), polytrimethylene terephthalate (PTT), polybutylene terephthalate (PBT), polyethylene isosorbide terephthalate (PEIT), polylactic acid (PLA), poly(L-lactic acid) (PLLA), poly(D-lactic acid) (PDLA), poly(D,L-lactic acid) (PDLLA), PLA stereocomplex (scPLA), polyhydroxy alkanoate (PHA), Poly(3-hydroxybutyrate) (P(3HB)/PHB), Poly(3-hydroxyvalerate) (P(3HV)/PHV), Poly(3- hydroxyhexanoate) (P(3HHx)), Poly(3-hydroxyoctanoate) (P(3HO)), Poly(3-hydroxydecanoate) (P(3HD)), Poly(3-hydroxybutyrate-co-3-hydroxyvalerate) (P(3HB-co-3HV)/PHBV), Poly(3- hydroxybutyrate-co-3-hydroxyhexanoate) (P(3HB-co-3HHx)/ (PHBHHx)), Poly(3- hydroxybutyrate-co-5-hydroxyvalerate) (PHB5HV), Poly(3-hydroxybutyrate-co-3- hydroxypropionate) (PHB3HP), Polyhydroxybutyrate-co-hydroxyoctonoate (PHBO), polyhydroxybutyrate-co-hydroxyoctadecanoate (PHBOd), Poly(3-hydroxybutyrate-co-3- hydroxyvalerate-co-4-hydroxybutyrate) (P(3HB-co-3HV-co-4HB)), polybutylene succinate (PBS), polybutylene succinate adipate (PBSA), polyhydroxyalkanoate (PHA), polybutylene adipate terephthalate (PBAT), polyethylene furanoate (PEF), Polycaprolactone (PCL), poly(ethylene adipate) (PEA), polyethylene naphthalate (PEN) and blends/mixtures thereof.

In a preferred embodiment, the plastic is selected from the list consisting of PE, PET or polyester, PVC, PS, PP, PU, Teflon, polybutyrate adipate terephthalate (PBAT), Polylactic acid (PLA), chlorinated polyethylene (CPE), acrylonitrile butadiene styrene (ABS), polycarbonate (PC), nylon, HDEP, LDEP, PE, and blends or mixtures thereof.

In another preferred embodiment, the composition of the invention also acts on plastics that might containing a polylactic acid bond to any of its forms and/or derivatives thereof, forming bonds with hydrocarbon polymers.

A "recycling process" or "complete recycling process" in relation to a plastic article refers to a process by which at least one polymer, monomer, etc of said plastic article is degraded to yield repolymerizable monomers and/or oligomers, which are advantageously retrieved in order to be reused.

In another preferred embodiment, the enzymatic extract included in the composition for degrading plastic products of the invention is obtained from a culture comprising at least three live *Trichoderma* species selected from the list consisting of *Trichoderma harzianum, Trichoderma viridae, Trichoderma longibratum,* and any variants thereof obtained in laboratory and identifies as T 22, Tr 115, Tr 116, KRL -AG 2 (Rifai), including holoforms as *Hypocrea* and *Podostroma,* wherein *T. longibratum* stump was transformed by mean of the exposition to radiation before being cultured with the other *Trichoderma* species. These at least three strains are cultivated all together, in a traditional bioreactor, on micronized and a slightly hydrated layer of oat as solid means of cultivation and fed with glucose-maltose as to obtain an aqueous solution rich in secretions but poor in Trichoderma biomass. The micronized oat is subjected to sterilization by controlled exposition to microwaves to avoid the degradation of the fructose contained in its germ.

The *Trichoderma longibratum* was previously treated with radiotherapy, particularly this strain was submitted to micro expositions to Cobalt for 50 generations, obtaining a stump with unique characteristics, resulting in an immune strain to the secretions released by species *Trichoderma harzianum* and *Trichoderma viridae.* These secretions are deactivated by the presence of metabolites that are released by the *Trichoderma longibratum* that change the pH of the medium allowing the absorption of the secretions by the oat. These enzymes are of the thermos-labile type and, in the oat are deactivated without degrading. When the culture has reached the maximum growth, the fungus-containing trays are harvested and immediately diluted in the substrate, which allows the fungus to grow more slowly and the dilution of the enzymes that could affect the fungus structure during the aging of the product.

According to the present invention, the three live Trichoderma species are cultured in the proportions: 10:20:70 or 99:05:05, respectively.

Thus, in a more preferred embodiment, the composition of the invention is obtained from a method comprising the following steps:
a) Initial sowing on trays containing the micronutrient oat, micro ionized sterile, and hydrated with at least three Trichoderma species, as mentioned above, in the form of original spores, in the following proportions: *Trichoderma harzianum* 50%, *Trichoderma viridae* 30% and *Trichoderma longibratum* (already irradiated) 20%.
b) Critical levels: the culture is handled in the following levels, pH 5, temperature 17°C to 22°C, with expositions of interrupted photo periods;
c) Ability of the nutrient to absorb, contain, and deliver (in aqueous solution) the volatile secretions, product of the mutual repulsion of the species Harziano and Viridae and the non-volatile secretions of *Trichoderma longibratum;*
d) The generated extract harvest implies the total collection of this by mechanical means, between days 4 and 7, its re-sowing is made from this point, using live material in all of its vegetative growth phases at 1% or more with respect to the already hydrated nutrient (micronized sterile and hydrated oat) over culture trays.

Therefore, the composition of the present invention as well as the method of obtaining thereof is disclosed in the document EP1384405 B1, which is included by reference to the present disclosure, particularly the information regarding the composition and the method of obtaining thereof. Thus, this document, EP1384405 B1 discloses that the Trichoderma species correspond to filament type fungus that are antagonistic with other fungus, including the same species, mainly as: *harzanium, viridae, polysporum, longibratum, koningii* and variations of these obtained in laboratory and identified as T 22, Tr 115, Tr 116, KRL-Ag 2 (Rifai), including holoforms as Hypocrea, Podostroma.

According to the invention, the term "enzymatic extract" designates active enzymes or enzyme-producing microorganisms, such as sporulating microorganisms, as well as combinations thereof, obtained from the culture of at least three live Trichoderma species selected from the list consisting of Trichoderma *harzianum, Trichoderma viridae, Trichoderma longibratum,* as previously mentioned. According to the invention, "enzymatic extract" preferably refers to enzymes obtained as previously mentioned.

In a preferred embodiment, the enzymatic extract of the composition of the invention might be concentrated, preferably in ultrapure water. In another particular embodiment of the present invention, the concentrated enzymatic extract to ultrapure water ratio is from about 1:1 to about 1:100, preferably, 1:10, 1:20; 1:30; 1:40; 1:50; 1:60; 1:70; 1:80; 1:90; 1:100.

In another preferred embodiment, the enzymatic extract comprises enzymes having between about 15 and about 100 kDa molecular weight; more particularly between about 15 and about 75 kDa molecular weight.

In another preferred embodiment, the enzymatic extract of the composition of the present invention comprises lytic enzymes from alpha/beta hydrolase superfamily selected from the list consisting of: carboxidase-transferase-hydrolases; transferase-hydrolase-carboxidases; hydrolase-carboxidase-transferases, or any combinations thereof. In a more preferred embodiment, the enzymatic extract comprises lytic enzymes carboximethyl cellulase, chitinase, b 1,3 gluconase, b xylosidase, xylanase; and a high concentration of 6 pentyl alphapyrone.

In another preferred embodiment, the enzymatic extract of the composition of the present invention was analyzed by chromatographic techniques, such as Liquid Chromatography with tandem Mass Spectrometry (LC-MS-MS) which combines the separation of proteins/peptides by liquid chromatography with their subsequent analysis by mass spectrometry followed by a bioinformatics study using specialized software and protein databases such as UniProt, which allows the identification of proteins. Thus, once the peptide sequences included in the extract of the invention are obtained, these sequences are matched against all the species belonging to the genus Trichoderma known in the UniProt sequence database, but further against the entire UniProt database comprising plants, fungi and bacteria peptides. Although UniProt database has been used in the present invention for the identification of the enzymes included in the enzymatic extract of the composition of the invention, any sequence database known by the skilled person is useful in the present invention.

**Table 1 shows the results obtained when the sequences of the enzymes included in the enzymatic extract of the invention are matched against bacteria peptides known in the UniProt sequence database:**

| **Prot. Hit number** | **Protein Accession Number (UniProt Database)** | **Protein description** | **Protein Score** | **Protein mass** |
|---|---|---|---|---|
| 1 | A0A2T4DA31 | IF rod domain-containing protein (Fragment) OS=Marivirga lumbricoides OX=1046115 GN=C9994_16990 PE=4 SV=1 | 408 | 21394 |
| 2 | A0A3S0EFW3 | Uncharacterized protein OS=Burkholderiales bacterium OX=1891238 GN=EKK47_01045 PE=4 SV=1 | 359 | 79865 |
| 3 | A0A6L9GQA3 | IF rod domain-containing protein (Fragment) OS=Rodentibacter pneumotropicus OX=758 GN=D3Z29_11520 PE=4 SV=1 | 296 | 39702 |
| 4 | A0A254NA27 | Uncharacterized protein OS=Pelomonas puraquae OX=431059 GN=CDO81_14340 PE=4 SV=1 | 286 | 49513 |
| 5 | A0A3S0CVM9 | Bifunctional metallophosphatase/5~-nucleotidase OS=Burkholderiales bacterium OX=1891238 GN=EKK47_13005 PE=3 SV=1 | 275 | 65971 |
| 6 | A0A370CMH6 | Keratin_2_tail domain-containing protein (Fragment) OS=Mycolicibacterium moriokaense OX=39691 GN=DVS77_34450 PE=4 SV=1 | 273 | 11104 |
| 7 | A0A246E3Y5 | Uncharacterized protein (Fragment) OS=Microbacterium sp. AISO3 OX=2002831 GN=CBF90_16015 PE=4 SV=1 | 236 | 49165 |
| 8 | A0A074TY77 | Thioredoxin (Fragment) OS=Microbacterium sp. SUBG005 OX=1504156 GN=trxA PE=4 SV=1 | 212 | 7649 |
| 9 | A0A522B7H2 | Uncharacterized protein OS=Aquabacterium sp. OX=1872578 GN=EP001_20905 PE=4 SV=1 | 209 | 79223 |
| 10 | M4Z814 | Porin OS=Bradyrhizobium oligotrophicum S58 OX=1245469 GN=S58_32640 PE=3 SV=1 | 190 | 53939 |
| 11 | A0A533J2R3 | Porin (Fragment) OS=Bradyrhizobium icense OX=1274631 GN=D1543_18045 PE=3 SV=1 | 190 | 57520 |
| 12 | A0A3N6NK80 | Porin OS=Bradyrhizobium sp. RP6 OX=2489596 GN=EHH60_02790 PE=3 SV=1 | 187 | 53991 |
| 13 | A0A3S0NJB3 | Porin OS=Bradyrhizobiaceae bacterium OX=2072420 GN=EKK32_06210 PE=3 SV=1 | 181 | 53897 |
| 14 | A0A4R6RI03 | Uncharacterized protein OS=Aquabacterium commune OX=70586 GN=EV672_102165 PE=4 SV=1 | 179 | 81132 |
| 15 | A0A254NFV5 | Uncharacterized protein OS=Pelomonas puraquae OX=431059 GN=CD081_01300 PE=4 SV=1 | 176 | 80055 |
| 16 | A0A0Q5CI29 | Methanol dehydrogenase OS=Methylobacterium sp. Leaf123 OX=1736264 GN=ASF53_20690 PE=3 SV=1 | 174 | 65155 |
| 17 | A0A023Z5G7 | Thioredoxin OS=Escherichia coli O145:H28 (strain RM12581) OX=1248823 GN=trxA PE=3 SV=1 | 172 | 11913 |
| 18 | A0A026UIT3 | Glutaredoxin OS=Escherichia coli O174:H8 str. 04-3038 OX=1446704 GN=BW70_24340 PE=4 SV=1 | 166 | 9793 |
| 19 | A0A263UQN7 | Actin, cytoplasmic 2 OS=Serratia marcescens OX=615 GN=CIG56_26370 PE=4 SV=1 | 163 | 36234 |
| 20 | A0A431KG07 | Acid phosphatase OS=Burkholderiales bacterium OX=1891238 GN=acpA PE=4 SV=1 | 158 | 58823 |
| 21 | A0A0X8CJW6 | Porin OS=Bradyrhizobium sp. CCGE-LA001 OX=1223566 GN=BCCGELA001_21530 PE=3 SV=1 | 155 | 54895 |
| 22 | A0A6P1RB92 | Porin OS=Bradyrhizobium sp. LCT2 OX=2493093 GN=EI171_23140 PE=3 SV=1 | 154 | 60661 |
| 23 | A0A431N951 | Porin (Fragment) OS=Rhizobiales bacterium OX=1909294 GN=EKK41_20090 PE=4 SV=1 | 136 | 36862 |
| 24 | A0A2D6YVJ4 | Glyceraldehyde-3-phosphate dehydrogenase OS=Phycisphaerae bacterium OX=2026778 GN=gap PE=3 SV=1 | 122 | 36768 |
| 25 | A0A1B1AH70 | Uncharacterized protein OS=Caulobacteraceae bacterium OTSz_A_272 OX=1759059 GN=ATE48_08070 PE=3 SV=1 | 121 | 107697 |
| 26 | A0A3S0DZJ0 | Uncharacterized protein OS=Rhizobiales bacterium OX=1909294 GN=EKK41_28395 PE=4 SV=1 | 119 | 40939 |
| 27 | A0A515KF77 | Porin OS=Tardiphaga sp. vice352 OX=2592816 GN=FNL55_03100 PE=3 SV=1 | 114 | 52035 |
| 28 | A0A0X1SH75 | Outer membrane protein assembly factor BamA OS=Methylobacterium sp. AMS5 OX=925818 GN=bamA PE=3 SV=1 | 113 | 93319 |
| 29 | A0A2D8N8K8 | Uncharacterized protein OS=Flavobacteriaceae bacterium OX=1871037 GN=CMC12_00545 PE=4 SV=1 | 111 | 60520 |
| 30 | A0A254NAE2 | Uncharacterized protein OS=Pelomonas puraquae OX=431059 GN=CDO81_12705 PE=4 SV=1 | 108 | 47800 |
| 31 | A0A069NNF5 | Thioredoxin OS=Caballeronia grimmiae OX=1071679 GN=BG57_15260 PE=3 SV=1 | 102 | 11952 |
| 32 | A0A1B1AK68 | Uncharacterized protein OS=Caulobacteraceae bacterium OTSz_A_272 OX=1759059 GN=ATE48_14050 PE=3 SV=1 | 100 | 106224 |
| 33 | A0A1B1AGC1 | Uncharacterized protein OS=Caulobacteraceae bacterium OTSz_A_272 OX=1759059 GN=ATE48_06545 PE=3 SV=1 | 98 | 89322 |
| 34 | A0A2E6NMV4 | PDZ domain-containing protein OS=Planctomycetes bacterium OX=2026780 GN=CMJ99_02990 PE=4 SV=1 | 94 | 47593 |
| 35 | A0A1Q4DZU9 | TonB-dependent receptor OS=Sphingomonas sp. 67-41 OX=1895850 GN=BGP17_17415 PE=3 SV=1 | 93 | 93207 |
| 36 | A0A4Q4ICU0 | Histone domain-containing protein (Fragment) OS=Sporolactobacillus sp. THM7-4 OX=2511170 GN=EWI07_14590 PE=4 SV=1 | 90 | 12549 |
| 37 | A0A1Z8Z9J9 | Oligoribonuclease OS=Cellvibrionales bacterium TMED79 OX=1986768 GN=orn PE=3 SV=1 | 88 | 20466 |
| 38 | A0A1B1AK07 | Uncharacterized protein OS=Caulobacteraceae bacterium OTSz_A_272 OX=1759059 GN=ATE48_13635 PE=4 SV=1 | 87 | 45249 |
| 39 | A0A011TJ34 | 60 kDa chaperonin OS=Aquamicrobium defluvii OX=69279 GN=groL PE=3 SV=1 | 82 | 58030 |
| 40 | A0A084ZTX2 | Thioredoxin OS=Trabulsiella guamensis ATCC 49490 OX=1005994 GN=trxA PE=3 SV=1 | 80 | 11887 |
| 41 | A0A066UTY4 | Thioredoxin OS=Vibrio fortis OX=212667 GN=trxA PE=3 SV=1 | 80 | 11742 |
| 42 | A0A2D5HGB1 | TonB-dependent receptor OS=Novosphingobium sp. OX=1874826 GN=CMH85_17015 PE=3 SV=1 | 78 | 93714 |
| 43 | A0A318D7Y7 | IF rod domain-containing protein OS=Kangiella spongicola OX=796379 GN=DL796_12550 PE=4 SV=1 | 77 | 52596 |
| 44 | A0A7C1AED6 | IMP cyclohydrolase (Fragment) OS=Desulfobacteraceae bacterium OX=2049433 GN=ENG35_01360 PE=4 SV=1 | 75 | 42868 |
| 45 | A0A0C5VUS7 | Glutamyl-tRNA synthetase OS=Siansivirga zeaxanthinifaciens CC-SAMT-1 OX=1454006 GN=AW14_03795 PE=4 SV=1 | 74 | 133135 |
| 46 | A0A1Q4E724 | TonB-dependent receptor OS=Sphingomonas sp. 67-41 OX=1895850 GN=BGP17_08415 PE=3 SV=1 | 74 | 100644 |
| 47 | A0A2K9PJT8 | Two-component system response regulator OS=Flavivirga eckloniae OX=1803846 GN=C1H87_00545 PE=4 SV=1 | 72 | 60283 |
| 48 | A0A2E3QKA2 | 3-oxoacyl-[acyl-carrier-protein] synthase 2 OS=Gammaproteobacteria bacterium OX=1913989 GN=fabF PE=3 SV=1 | 72 | 43115 |
| 49 | A0A402BJF3 | Lacl family transcriptional regulator OS=Dictyobacter alpinus OX=2014873 GN=KDA_69590 PE=4 SV=1 | 72 | 48475 |
| 50 | A0A1B1ALA9 | Flagellin OS=Caulobacteraceae bacterium OTSz_A_272 OX=1759059 GN=ATE48_16125 PE=3 SV=1 | 71 | 42300 |
| 51 | A0A2H5YIZ6 | Uncharacterized protein OS=bacterium HR24 OX=2035419 GN=HRbin24_01473 PE=4 SV=1 | 71 | 20743 |
| 52 | A0A6L9HM42 | IF rod domain-containing protein (Fragment) OS=Clostridiaceae bacterium OX=1898204 GN=D3Z50_22930 PE=4 SV=1 | 71 | 8440 |
| 53 | A0A7W4HJ08 | Uncharacterized protein (Fragment) OS=Candidatus Saccharibacteria bacterium OX=2026720 GN=HG452_002535 PE=4 SV=1 | 70 | 98668 |
| 54 | A0A554K3V5 | Ribonuclease Y OS=Parcubacteria group bacterium Gr01-1014_30 OX=2017182 GN=rny PE=3 SV=1 | 70 | 56220 |
| 55 | A0A2V9DEB8 | MBL fold metallo-hydrolase OS=Acidobacteria bacterium OX=1978231 GN=DMG31_12590 PE=4 SV=1 | 70 | 34139 |
| 56 | A0A431K277 | Porin OS=Burkholderiales bacterium OX=1891238 GN=EKK47_22090 PE=4 SV=1 | 70 | 39139 |
| 57 | A0A2V8RN55 | DUF58 domain-containing protein OS=Acidobacteria bacterium OX=1978231 GN=DMF62_10780 PE=4 SV=1 | 69 | 53766 |
| 58 | A0A379PNV6 | Uncharacterized protein OS=Rhodococcus gordoniae OX=223392 GN=NCTC13296_04175 PE=4 SV=1 | 68 | 15902 |
| 59 | A0A257HNE1 | TonB-dependent receptor OS=Alphaproteobacteria bacterium PA1 OX=2015569 GN=CFE27_07940 PE=3 SV=1 | 68 | 95778 |
| 60 | A0A178IQF7 | Uncharacterized protein OS=Opitutaceae bacterium TSB47 OX=1184151 GN=AW736_03085 PE=3 SV=1 | 67 | 380964 |
| 61 | A0A1V0A5H8 | Copper oxidase OS=Nonomuraea sp. ATCC 55076 OX=1909395 GN=BKM31_31970 PE=4 SV=1 | 66 | 51717 |
| 62 | A0A4Q5QZA4 | Peptidylprolyl isomerase (Fragment) OS=Chitinophagaceae bacterium OX=1869212 GN=E0004_31340 PE=4 SV=1 | 66 | 61230 |
| 63 | R6EA41 | Uncharacterized protein OS=Prevotella sp. CAG:1320 OX=1262922 GN=BN487_00502 PE=4 SV=1 | 66 | 36975 |
| 64 | A0A4Q7YGW2 | ATP-dependent Clp protease proteolytic subunit OS=Streptomyces sp. BK022 OX=2512123 GN=EV284_3386 PE=3 SV=1 | 65 | 10595 |
| 65 | F8E1Y8 | D-alanyl-D-alanine carboxypeptidase / D-alanyl-D-alanine-endopeptidase OS=Corynebacterium resistens (strain DSM 45100 / JCM 12819 / GTC 2026 / SICGH 158) OX=662755 GN=dac PE=3 SV=1 | 65 | 46888 |
| 66 | A0A6F9ZXX8 | DNA primase OS=Candidatus Uhrbacteria bacterium OX=2053634 GN=dnaG PE=3 SV=1 | 65 | 68877 |
| 67 | A0A086EK87 | Ribosomal RNA large subunit methyltransferase G OS=Pectobacterium brasiliense OX=180957 GN=rlmG PE=3 SV=1 | 65 | 42630 |
| 68 | A0A2K4IRP5 | Histidine kinase OS=Pseudomonas sp. FW507-12TSA OX=2075552 GN=C1889_30350 PE=4 SV=1 | 65 | 100421 |
| 69 | U1IHA6 | PBP_domain domain-containing protein OS=Bradyrhizobium sp. DFCI-1 OX=1230476 GN=C207_03704 PE=4 SV=1 | 64 | 66693 |
| 70 | A0A1I3BQ73 | Inositol hexakisphosphate OS=Selenomonas ruminantium OX=971 GN=SAMN04487861_101149 PE=4 SV=1 | 64 | 39918 |
| 71 | A0A2E9R5S6 | Uncharacterized protein OS=Deltaproteobacteria bacterium OX=2026735 GN=CL920_35700 PE=4 SV=1 | 63 | 58159 |
| 72 | A0A1B1AE71 | OmpA-like domain-containing protein OS=Caulobacteraceae bacterium OTSz_A_272 OX=1759059 GN=ATE48_02380 PE=4 SV=1 | 62 | 38580 |
| 73 | A0A7X0DBT4 | Aldehyde dehydrogenase (NAD+) OS=Rhizobium flavum OX=1335061 GN=HNQ75_000179 PE=4 SV=1 | 62 | 51235 |
| 74 | A0A2N3FR94 | Transcription elongation factor GreA OS=Actinobacteria bacterium HGW-Actinobacteria-5 OX=2013650 GN=greA PE=3 SV=1 | 61 | 18181 |
| 75 | A0A165PJL3 | Uncharacterized protein OS=Pseudovibrio sp. W74 OX=1735584 GN=PsW74_03732 PE=4 SV=1 | 60 | 40913 |
| 76 | A0A6G6YEM7 | MFS transporter OS=Chryseobacterium sp. POL2 OX=2713414 GN=G6R40_01035 PE=4 SV=1 | 60 | 47761 |
| 77 | A0A426QNA7 | DNA-directed RNA polymerase subunit beta OS=Guyparkeria sp. SCN-R1 OX=2341113 GN=rpoB PE=3 SV=1 | 60 | 154824 |
| 78 | A0A1 F4MZK2 | Uncharacterized protein OS=Burkholderiales bacterium RIFCSPLOWO2_12_FULL_64_99 OX=1797568 GN=A3G29_03535 PE=4 SV=1 | 59 | 36475 |
| 79 | A0A344KZV7 | (2Fe-2S)-binding protein OS=Amycolatopsis albispora OX=1804986 GN=A4R43_01345 PE=4 SV=1 | 59 | 17340 |
| 80 | A0A1Q3H8B5 | Uncharacterized protein OS=Archangium sp. Cb G35 OX=1920190 GN=BO221_45415 PE=4 SV=1 | 59 | 134377 |
| 81 | A0A0M8KBD0 | Alpha-amylase OS=Ardenticatena maritima OX=872965 GN=ARMA_2682 PE=3 SV=1 | 59 | 66196 |
| 82 | A0A0D6JHV1 | Uncharacterized protein OS=Candidatus Filomicrobium marinum OX=1608628 GN=YBN1229_v1_2997 PE=4 SV=1 | 59 | 45032 |
| 83 | A0A7X8ZAB3 | Sugar ABC transporter substrate-binding protein OS=Thermoanaerobacteraceae bacterium OX=2100788 GN=GX892_07115 PE=4 SV=1 | 59 | 33996 |
| 84 | A0A0K6IUT1 | Penicillin-binding protein 2 OS=Tepidiphilus thermophilus OX=876478 GN=Ga0061068_10477 PE=4 SV=1 | 59 | 68567 |
| 85 | A0A0Q5V4K7 | LexA_DNA_bind domain-containing protein OS=Brevundimonas sp. Leaf363 OX=1736353 GN=ASG17_07690 PE=4 SV=1 | 59 | 12957 |
| 86 | A0A4R8DEV0 | DUF2961 family protein OS=Dinghuibacter silviterrae OX=1539049 GN=EDB95_3796 PE=4 SV=1 | 59 | 43218 |

**Table 2 shows the results obtained when the sequences of the enzymes included in the enzymatic extract of the invention are matched against fungi peptides known in the UniProt sequence database.**

| **Prot. Hit number** | **Protein Accession Number (UniProt Database)** | **Protein description** | **Protein Score** | **Protein mass** |
|---|---|---|---|---|
| 1 | A0A086T4A0 | Glucan endo-1,3-beta-glucosidase-like protein OS=Acremonium chrysogenum (strain ATCC 11550 / CBS 779.69 / DSM 880 / JCM 23072 / IMI 49137) OX=857340 GN=ACRE_050450 PE=4 SV=1 | 67 | 45789 |
| 2 | A0A061HIF9 | Glyceraldehyde-3-phosphate dehydrogenase OS=Blumeria graminis f. sp. tritici 96224 OX=1268274 GN=BGT96224_712 PE=3 SV=1 | 66 | 36831 |
| 3 | G0RXZ8 | Glyceraldehyde-3-phosphate dehydrogenase OS=Chaetomium thermophilum (strain DSM 1495 / CBS 144.50 / IMI 039719) OX=759272 GN=CTHT 0004880 PE=3 SV=1 | 66 | 36618 |
| 4 | A0A137P2C3 | Tubulin beta chain OS=Conidiobolus coronatus (strain ATCC 28846 / CBS 209.66 / NRRL 28638) OX=796925 GN=CONCODRAFT_79440 PE=3 SV=1 | 65 | 51183 |
| 5 | A0A060SXR3 | Actin OS=Blastobotrys adeninivorans OX=409370 GN=GNL VRS02_ARAD1 A06666g PE=3 SV=1 | 62 | 40351 |
| 6 | A0A167C144 | Actin OS=Sugiyamaella lignohabitans OX=796027 GN=ACT1 PE=3 SV=1 | 62 | 40374 |
| 7 | A0A5N7AZH7 | BUD22-domain-containing protein OS=Aspergillus bertholletiae OX=1226010 GN=BDV26DRAFT_284043 PE=3 SV=1 | 61 | 77419 |
| 8 | A0A4Y9Y4H9 | USP domain-containing protein OS=Fomitopsis rosea OX=34475 GN=EVJ58_g7480 PE=4 SV=1 | 60 | 145391 |
| 9 | A0A2T7A1T5 | Uncharacterized protein OS=Tuber borchii OX=42251 GN=B9Z19DRAFT_1121932 PE=4 SV=1 | 60 | 105312 |
| 10 | A0A060S977 | WLM domain-containing protein OS=Pycnoporus cinnabarinus OX=5643 GN=BN946_scf185000.g91 PE=4 SV=1 | 60 | 47454 |
| 11 | A0A0C2TM17 | Uncharacterized protein OS=Amanita muscaria Koide BX008 OX=946122 GN=M378DRAFT 8869 PE=4 SV=1 | 60 | 70249 |
| 12 | A0A3M7NED5 | Kinase (Fragment) OS=Chaetothyriales sp. CBS 132003 OX=2249419 GN=DV737_g584 PE=3 SV=1 | 58 | 59017 |
| 13 | A0A1G4J087 | DNA polymerase OS=Lachancea dasiensis OX=1072105 GN=LADA 0C08966G PE=3 SV=1 | 58 | 168398 |
| 14 | A0A175WFT5 | D-lactate dehydrogenase [cytochrome], mitochondrial OS=Madurella mycetomatis OX=100816 GN=MMYC01_201669 PE=3 SV=1 | 58 | 63134 |
| 15 | W9Y7T2 | Uncharacterized protein OS=Capronia coronata CBS 617.96 OX=1182541 GN=A1O1_08849 PE=3 SV=1 | 56 | 37133 |
| 16 | A0A0B7K534 | CAP-Gly domain-containing protein OS=Bionectria ochroleuca OX=29856 GN=BN869_000006182_1 PE=3 SV=1 | 55 | 142031 |
| 17 | A0A165ZNR0 | TFIID-18kDa-domain-containing protein OS=Peniophora sp. CONT OX=1314672 GN=PENSPDRAFT_641671 PE=4 SV=1 | 54 | 25500 |
| 18 | A0A0L0HB80 | Uncharacterized protein OS=Spizellomyces punctatus (strain DAOM BR117) OX=645134 GN=SPPG_06967 PE=4 SV=1 | 54 | 70770 |
| 19 | A0A150V7Q7 | Uncharacterized protein OS=Acidomyces richmondensis BFW OX=766039 GN=M433DRAFT_142827 PE=4 SV=1 | 54 | 26854 |
| 20 | A0A7C8IKG9 | Uncharacterized protein OS=Massariosphaeria phaeospora OX=100035 GN=BDV95DRAFT_486025 PE=4 SV=1 | 54 | 77139 |
| 21 | A0A7C8PT02 | Uncharacterized protein OS=Arthrobotrys oligospora OX=2813651 GN=TWF751_001435 PE=4 SV=1 | 53 | 50541 |
| 22 | A0A086J133 | Uncharacterized protein OS=Nematocida sp. 1 ERTm6 OX=1138374 GN=NESG_01839 PE=4 SV=1 | 53 | 58575 |
| 23 | A0A077WXM8 | Uncharacterized protein OS=Lichtheimia ramosa OX=688394 GN=LRAMOSA04595 PE=4 SV=1 | 52 | 92332 |
| 24 | A0A0C2WW95 | Non-specific serine/threonine protein kinase OS=Serendipita vermifera MAFF 305830 OX=933852 GN=M408DRAFT 21934 PE=3 SV=1 | 51 | 265268 |
| 25 | A0A367JG23 | Uncharacterized protein OS=Rhizopus stolonifer OX=4846 GN=CU098 004980 PE=4 SV=1 | 50 | 78572 |
| 26 | A0A2S5B4C6 | Uncharacterized protein OS=Rhodotorula taiwanensis OX=741276 GN=BMF94_5380 PE=4 SV=1 | 49 | 70846 |
| 27 | W6YFU2 | NmrA domain-containing protein OS=Bipolaris zeicola 26-R-13 OX=930089 GN=COCCADRAFT_93759 PE=4 SV=1 | 49 | 37805 |
| 28 | A0A0C3SAF0 | Uncharacterized protein OS=Phlebiopsis gigantea 11061_1 CR5-6 OX=745531 GN=PHLGIDRAFT_106286 PE=3 SV=1 | 49 | 94366 |
| 29 | A0A0L0VIW6 | Uncharacterized protein OS=Puccinia striiformis f. sp. tritici PST-78 OX=1165861 GN=PSTG_07538 PE=4 SV=1 | 48 | 13622 |
| 30 | A0A0A8L343 | WGS project CCBQ000000000 data, contig 00098 OS=Kluyveromyces dobzhanskii CBS 2104 OX=1427455 GN=KLDO_g1775 PE=3 SV=1 | 48 | 123400 |
| 31 | A0A014N9P0 | Bifunctional trehalose-6-phosphate synthase/HAD hydrolase subfamily IIB (Glycosyl transferase family 20) OS=Metarhizium robertsii OX=568076 GN=X797_009368 PE=3 SV=1 | 47 | 101511 |
| 32 | A0A0B7NBU7 | UVR domain-containing protein OS=Parasitella parasitica OX=35722 GN=PARPA_10264.1 scaffold 40051 PE=4 SV=1 | 47 | 97928 |
| 33 | A0A4Z1P540 | UBC core domain-containing protein OS=Venturia nashicola OX=86259 GN=E6075_ATG09702 PE=4 SV=1 | 46 | 133471 |
| 34 | A0A068RL11 | Ring finger protein OS=Lichtheimia corymbifera JMRC:FSU:9682 OX=1263082 GN=LCOR_02112.1 PE=4 SV=1 | 46 | 62667 |
| 35 | A0A194SBF8 | Mediator complex subunit 12 OS=Rhodotorula graminis (strain WP1) OX=578459 GN=RHOBADRAFT_51600 PE=3 SV=1 | 46 | 204497 |
| 36 | A0A061AJS9 | RHTO0S01e02190g1_1 OS=Rhodosporidium toruloides OX=5286 GN=RHTC0S_01e02190g PE=4 SV=1 | 45 | 75323 |
| 37 | A0A4Z0YW47 | Uncharacterized protein OS=Xylaria hypoxylon OX=37992 GN=E0Z10_g7081 PE=4 SV=1 | 45 | 451562 |
| 38 | H2AZ52 | PRELI/MSF1 domain-containing protein OS=Kazachstania africana (strain ATCC 22294 / BCRC 22015 / CBS 2517 / CECT 1963 / NBRC 1671 / NRRL Y-8276) OX=1071382 GN=KAFR0H01990 PE=4 SV=1 | 45 | 21185 |
| 39 | A0A5N5LJX3 | H/ACA ribonucleoprotein complex subunit OS=Coniochaeta sp. 2T2.1 OX=1571157 GN=GE09DRAFT_248366 PE=3 SV=1 | 45 | 22161 |
| 40 | A0A095C687 | Serine hydroxymethyltransferase OS=Cryptococcus gattii serotype B (strain R265) OX=294750 GN=CNBG_1047 PE=3 SV=1 | 45 | 54688 |
| 41 | A0A1J7J0V1 | Uncharacterized protein OS=Coniochaeta ligniaria NRRL 30616 OX=1408157 GN=CONLIGDRAFT_590642 PE=4 SV=1 | 45 | 70766 |
| 42 | A0A3E2HBN2 | Uncharacterized protein (Fragment) OS=Scytalidium lignicola OX=5539 GN=B7463 g5471 PE=4 SV=1 | 44 | 16688 |
| 43 | A0A135TYM1 | RecF/RecN/SMC N terminal domain-containing protein OS=Colletotrichum simmondsii OX=703756 GN=CSIM01_13085 PE=3 SV=1 | 44 | 133191 |
| 44 | A0A1G4M912 | LAFE_0B12266g 1_1 OS=Lachancea fermentati OX=4955 GN=LAFE_0B12266G PE=4 SV=1 | 44 | 136796 |
| 45 | A0A194XRZ2 | Uncharacterized protein OS=Phialocephala scopiformis OX=149040 GN=LY89DRAFT_664605 PE=4 SV=1 | 44 | 95602 |
| 46 | F4NV28 | Uncharacterized protein (Fragment) OS=Batrachochytrium dendrobatidis (strain JAM81 / FGSC 10211) OX=684364 GN=BATDEDRAFT_1311 PE=3 SV=1 | 43 | 50920 |
| 47 | A0A0U5GKF6 | Uncharacterized protein OS=Aspergillus calidoustus OX=454130 GN=ASPCAL15021 PE=4 SV=1 | 43 | 39174 |
| 48 | A0A0D2AL24 | Uncharacterized protein OS=Verruconis gallopava OX=253628 GN=PV09 08564 PE=4 SV=1 | 43 | 60602 |
| 49 | A0A1B8DMC1 | DAO domain-containing protein OS=Pseudogymnoascus sp. 23342-1-I1 OX=1524831 GN=VE03_10279 PE=4 SV=1 | 42 | 51203 |
| 50 | A0A1B8AEZ6 | Uncharacterized protein OS=Fusarium poae OX=36050 GN=FPOA 10769 PE=4 SV=1 | 42 | 69092 |
| 51 | A0A559KU05 | Putative 30S ribosomal protein S17-like protein OS=Fusarium oxysporum f. sp. cubense OX=61366 GN=Focb16_v015260 PE=4 SV=1 | 42 | 112029 |
| 52 | A0A7C8IKI7 | Nascent polypeptide-associated complex subunit beta OS=Xylaria multiplex OX=323545 GN=GQX73, g7606 PE=3 SV=1 | 42 | 172368 |
| 53 | A0A1E1JY17 | Uncharacterized protein OS=Rhynchosporium agropyri OX=914238 GN=RAG0_01670 PE=4 SV=1 | 42 | 72989 |
| 54 | A0A067MMM4 | Uncharacterized protein OS=Botryobasidium botryosum FD-172 SS1 OX=930990 GN=BOTBODRAFT_66891 PE=4 SV=1 | 42 | 20479 |
| 55 | W4KCR3 | Uds1 domain-containing protein OS=Heterobasidion irregulare (strain TC 32-1) OX=747525 GN=HETIRDRAFT_155113 PE=4 SV=1 | 42 | 98768 |
| 56 | A0A317X534 | Uncharacterized protein OS=Aspergillus sclerotioniger CBS 115572 OX=1450535 GN=BO94DRAFT_342319 PE=4 SV=1 | 41 | 185992 |
| 57 | A0A6A5T961 | Uncharacterized protein OS=Byssothecium circinans OX=147558 GN=CC80DRAFT_511543 PE=4 SV=1 | 41 | 19330 |
| 58 | A0A1Y3NMF8 | Uncharacterized protein OS=Piromyces sp. (strain E2) OX=73868 GN=PIROE2DRAFT_3307 PE=4 SV=1 | 40 | 57780 |
| 59 | A0A074WBG6 | Uncharacterized protein OS=Aureobasidium namibiae CBS 147.97 OX=1043004 GN=M436DRAFT _57276 PE=4 SV=1 | 40 | 51396 |
| 60 | A0A015IG92 | Histone H2A OS=Rhizophagus irregularis (strain DAOM 197198w) OX=1432141 GN=RirG_218880 PE=3 SV=1 | 40 | 15806 |
| 61 | A0A0B1PBJ6 | Putative hsp90-like protein OS=Uncinula necator OX=52586 GN=EV44 g0807 PE=4 SV=1 | 40 | 238140 |
| 62 | A0A197K5E0 | p-loop containing nucleoside triphosphate hydrolase protein OS=Linnemannia elongata AG-77 OX=1314771 GN=K457DRAFT 70789 PE=4 SV=1 | 40 | 50142 |
| 63 | A0A146IHB3 | Uncharacterized protein OS=Mycena chlorophos OX=658473 GN=MCHLO 15311 PE=4 SV=1 | 40 | 13980 |
| 64 | A0A0L6WIF9 | Uncharacterized protein OS=Termitomyces sp. J132 OX=1306850 GN=J132_05888 PE=4 SV=1 | 40 | 200556 |
| 65 | A0A0F4YWQ6 | Glucoamylase OS=Rasamsonia emersonii CBS 393.64 OX=1408163 GN=T310_3423 PE=3 SV=1 | 40 | 65712 |
| 66 | A0A0F0I4J0 | Exportin 1-like protein OS=Aspergillus parasiticus (strain ATCC 56775 / NRRL 5862 / SRRC 143 / SU-1) OX=1403190 GN=P875_00034425 PE=4 SV=1 | 39 | 142158 |
| 67 | M3A4G9 | Uncharacterized protein OS=Pseudocercospora fijiensis (strain CIRAD86) OX=383855 GN=MYCFIDRAFT_171838 PE=4 SV=1 | 39 | 22490 |
| 68 | A0A1X0QW89 | Sister chromatid cohesion protein OS=Rhizopus microsporus var. microsporus OX=86635 GN=BCV72DRAFT_295446 PE=3 SV=1 | 39 | 129758 |
| 69 | A0A0H2RP11 | S-adenosyl-L-methionine-dependent methyltransferase OS=Schizopora paradoxa OX=27342 GN=SCHPADRAFT_355507 PE=4 SV=1 | 39 | 30579 |
| 70 | A0A1X0R593 | CAF1-domain-containing protein OS=Rhizopus microsporus var. microsporus OX=86635 GN=BCV72DRAFT_206063 PE=3 SV=1 | 38 | 86272 |
| 71 | A0A010QSL8 | ATP synthase subunit beta OS=Colletotrichum fioriniae PJ7 OX=1445577 GN=CFIO01_10693 PE=3 SV=1 | 38 | 55448 |
| 72 | A0A0B7FG10 | Uncharacterized protein OS=Thanatephorus cucumeris (strain AG1-IB / isolate 7/3/14) OX=1108050 GN=RSOLAG1IB_01131 PE=4 SV=1 | 37 | 37335 |
| 73 | A0A433PWN9 | Diadenosine tetraphosphate synthetase OS=Endogone sp. FLAS-F59071 OX=2340872 GN=BC937DRAFT_91225 PE=3 SV=1 | 37 | 74891 |
| 74 | A0A0U5GRU7 | NmrA domain-containing protein OS=Aspergillus calidoustus OX=454130 GN=ASPCAL04710 PE=3 SV=1 | 37 | 41129 |
| 75 | A0A093UTQ5 | Molybdopterin molybdenumtransferase OS=Talaromyces marneffei PM1 OX=1077442 GN=GQ26_0360500 PE=3 SV=1 | 37 | 51828 |
| 76 | A0A2T9YA77 | Uncharacterized protein OS=Smittium simulii OX=133385 GN=BB561_005494 PE=4 SV=1 | 37 | 208279 |
| 77 | J3K5H1 | Uncharacterized protein OS=Coccidioides immitis (strain RS) OX=246410 GN=CIMG_08421 PE=4 SV=2 | 36 | 31354 |
| 78 | A0A5C3EHF1 | Related to transport protein USO1 OS=Ustilago trichophora OX=86804 GN=UTRI 05943 PE=4 SV=1 | 36 | 233173 |
| 79 | A0A0J6F6I3 | Histone acetyltransferase OS=Coccidioides posadasii RMSCC 3488 OX=454284 GN=CPAG_02126 PE=3 SV=1 | 36 | 153327 |
| 80 | A0A1R1XNE7 | GYF domain-containing protein mpd2 OS=Smittium culicis OX=133412 GN=AYI69_g7951 PE=4 SV=1 | 36 | 98155 |
| 81 | A0A2I1CS73 | Beta-ketoacyl-[acyl-carrier-protein] synthase I OS=Aspergillus campestris IBT 28561 OX=1392248 GN=P168DRAFT_243687 PE=3 SV=1 | 36 | 199194 |
| 82 | A0A6A5XNZ1 | PLP-dependent transferase OS=Aaosphaeria arxii CBS 175.79 OX=1450172 GN=BU24DRAFT_214963 PE=3 SV=1 | 35 | 47828 |
| 83 | A0A0A2JS80 | ATPase, AAA-type, core OS=Penicillium expansum OX=27334 GN=PEX2_067690 PE=3 SV=1 | 34 | 68092 |
| 84 | A0A2S4V1U3 | Uncharacterized protein OS=Puccinia striiformis OX=27350 GN=PSHT _11626 PE=4 SV=1 | 34 | 178684 |
| 85 | A0A0C9MFB0 | Centrosomal protein of 104 kDa-like OS=Mucor ambiguus OX=91626 GN=MAM1_0107d05460 PE=4 SV=1 | 34 | 102906 |
| 86 | A0A5N5QMS2 | NudC domain-containing protein 1 OS=Ceratobasidium theobromae OX=1582974 GN=CTheo_3452 PE=4 SV=1 | 34 | 67314 |
| 87 | A0A061HBY7 | Protein kinase domain-containing protein OS=Anthracocystis flocculosa PF-1 OX=1277687 GN=PFL1_02657 PE=4 SV=1 | 34 | 85579 |
| 88 | A0A068RXC5 | Protein-tyrosine-phosphatase OS=Lichtheimia corymbifera JMRC:FSU:9682 OX=1263082 GN=LCOR 04949.1 PE=4 SV=1 | 34 | 80961 |
| 89 | A0A1Y1UC25 | Chromatin associated protein KTI12 OS=Kockovaella imperatae OX=4999 GN=BD324DRAFT_583192 PE=4 SV=1 | 33 | 31790 |
| 90 | A0A4Q4XGX8 | Uncharacterized protein OS=Monosporascus sp. 5C6A OX=2211642 GN=DL771_010709 PE=4 SV=1 | 33 | 80606 |
| 91 | A0A0L8RPX2 | Pre-mRNA-processing protein 45 OS=Saccharomyces eubayanus OX=1080349 GN=D149_0030 PE=3 SV=1 | 33 | 42229 |
| 92 | U1HWM0 | Uncharacterized protein OS=Endocarpon pusillum (strain Z07020 / HMAS-L-300199) OX=1263415 GN=EPUS_06185 PE=4 SV=1 | 33 | 53383 |
| 93 | A0A5M3Z343 | Nuclear architecture-related protein 1 OS=Aspergillus terreus OX=33178 GN=ATETN484_0006028000 PE=3 SV=1 | 32 | 65093 |
| 94 | A0A067N3I3 | Uncharacterized protein OS=Botryobasidium botryosum FD-172 SS1 OX=930990 GN=BOTBODRAFT_205941 PE=4 SV=1 | 32 | 9933 |
| 95 | I2K1V1 | Uncharacterized protein OS=Brettanomyces bruxellensis AWRI1499 OX=1124627 GN=AWRI1499_0815 PE=4 SV=2 | 32 | 39255 |
| 96 | A0A5N6UT32 | Class II histone deacetylase complex subunits 2 and 3-domain-containing protein OS=Aspergillus tamarii OX=41984 GN=BDV40DRAFT_176101 PE=4 SV=1 | 31 | 128985 |
| 97 | A0A1T3CS30 | Uncharacterized protein OS=Trichoderma guizhouense OX=1491466 GN=A0028_0021130 PE=4 SV=1 | 31 | 54018 |
| 98 | A0A395N2T1 | Heterokaryon incompatibility protein or allele OS=Fusarium flagelliforme OX=2675880 GN=FIE12Z_1343 PE=4 SV=1 | 31 | 60184 |
| 99 | A0A024RXF1 | HET-domain-containing protein OS=Hypocrea jecorina (strain ATCC 56765 / BCRC 32924 / NRRL 11460 / Rut C-30) OX=1344414 GN=M419DRAFT 26737 PE=4 SV=1 | 30 | 98363 |
| 100 | A0A067QC61 | DNA polymerase OS=Jaapia argillacea MUCL 33604 OX=933084 GN=JAAARDRAFT_53942 PE=3 SV=1 | 30 | 173295 |
| 101 | A0A1V1SZD2 | Uncharacterized protein OS=fungal sp. No.14919 OX=1813822 GN=ANO14919_033230 PE=4 SV=1 | 30 | 30940 |
| 102 | Q0UNL0 | Uncharacterized protein OS=Phaeosphaeria nodorum (strain SN15 / ATCC MYA-4574 / FGSC 10173) OX=321614 GN=JI435_066540 PE=4 SV=1 | 29 | 133351 |
| 103 | A0A066WYF9 | Uncharacterized protein OS=Colletotrichum sublineola OX=1173701 GN=CSUB01_11754 PE=4 SV=1 | 29 | 50733 |
| 104 | A0A136IXH0 | p-loop containing nucleoside triphosphate hydrolase protein OS=Microdochium bolleyi OX=196109 GN=Micbo1qcDRAFT_164934 PE=4 SV=1 | 29 | 72679 |
| 105 | A0A2I0RS63 | DNA replication checkpoint protein teI2 OS=Cercospora zeina OX=348901 GN=BST61_czeina50g000040 PE=3 SV=1 | 29 | 111183 |
| 106 | A0A0B1PFH2 | Putative integral membrane channel protein OS=Uncinula necator OX=52586 GN=EV44_g6377 PE=4 SV=1 | 28 | 115748 |
| 107 | A0A1L9UA41 | MFS domain-containing protein OS=Aspergillus brasiliensis (strain CBS 101740 / IMI 381727 / IBT 21946) OX=767769 GN=ASPBRDRAFT_133237 PE=4 SV=1 | 28 | 55805 |
| 108 | A0A1Y1VKP8 | CLIP1_ZNF domain-containing protein OS=Piromyces finnis OX=1754191 GN=BCR36DRAFT_453294 PE=4 SV=1 | 28 | 38640 |
| 109 | A0A017SJA0 | Putative flavin-binding monooxygenase OS=Aspergillus ruber CBS 135680 OX=1388766 GN=EURHEDRAFT_452328 PE=4 SV=1 | 28 | 67644 |
| 110 | A0A2H3TNL4 | Uncharacterized protein OS=Fusarium oxysporum OX=5507 GN=FRV6_08963 PE=4 SV=1 | 28 | 58410 |
| 111 | A0A178AI27 | Kinase-like protein OS=Stagonospora sp. SRC1IsM3a OX=765868 GN=IQ06DRAFT 350909 PE=4 SV=1 | 27 | 73463 |
| 112 | A0A2W1EPX6 | RbsK Sugar kinase ribokinase family OS=Pyrenophora tritici-repentis OX=45151 GN=A1F97_08195 PE=4 SV=1 | 27 | 39774 |
| 113 | A0A423X1B4 | Protein BCP1 OS=Valsa malicola OX=356882 GN=VMCG_02578 PE=3 SV=1 | 27 | 32402 |
| 114 | A0A2N5U044 | Uncharacterized protein OS=Puccinia coronata f. sp. avenae OX=200324 GN=PCANC_21477 PE=4 SV=1 | 27 | 14088 |
| 115 | A0A060TIV6 | ARAD1D41448p OS=Blastobotrys adeninivorans OX=409370 GN=GNLVRS02_ARAD1D41448g PE=3 SV=1 | 27 | 45337 |
| 116 | A0A061AQU4 | CYFA0S01e16424g1_1 OS=Cyberlindnera fabianii OX=36022 GN=CYFA0S_01e16424g PE=3 SV=1 | 26 | 87583 |
| 117 | A0A2J6SCW6 | Phosphoglycerate kinase OS=Hyaloscypha variabilis F OX=1149755 GN=L207DRAFT_477088 PE=3 SV=1 | 26 | 44469 |
| 118 | A0A135SGK3 | Uncharacterized protein OS=Colletotrichum simmondsii OX=703756 GN=CSIM01_01953 PE=4 SV=1 | 26 | 141662 |
| 119 | A0A4Y9ZZ24 | Phosphopyruvate hydratase OS=Hericium alpestre OX=135208 GN=EWM64 g3923 PE=3 SV=1 | 26 | 48346 |
| 120 | J5J6R0 | Transcription regulator PAB1642 OS=Beauveria bassiana (strain ARSEF 2860) OX=655819 GN=BBA 08847 PE=4 SV=1 | 26 | 29295 |
| 121 | A0A136JBR2 | Glucose inhibited division protein A-domain-containing protein OS=Microdochium bolleyi OX=196109 GN=Micbo1qcDRAFT_132610 PE=3 SV=1 | 26 | 144451 |
| 122 | A0A0C3G348 | WD_REPEATS_REGION domain-containing protein OS=Piloderma croceum (strain F 1598) OX=765440 GN=PILCRDRAFT_811147 PE=4 SV=1 | 25 | 106060 |

As can be seen from the results shown in **Tables 1 and 2,** no proteins with homology to any species of Trichoderma have been detected, however, proteins with homology to proteins of wheat (*Triticum aestivum*), barley (*Hordeum vulgare*), oats (*Avena sativa*) and different bacteria species of the genus Lactobacillus (*L*. *brevis and L. plantarum*)*,* have been found. Among the proteins detected and found in the enzymatic extract of the present invention, the proteins shown in **Table 3** are the most important:

**Table 3. Enzymes included in the enzymatic extract of the present invention**

| **Accession Number (Uniprot database)** | **Name** | **Specie** | **PEP Score** | **Peptides** | **Unique Peptides** | **Abundance** |
|---|---|---|---|---|---|---|
| Q8L5C6 | Xylanase inhibitor protein | *Triticum aestivum* | 60.201 | 16 | 16 | 2.08* 109 |
| Q03TX3 | Xylose isomerase | *Lactobacillus brevis* | 71.541 | 21 | 15 | 1.75*108 |
| P11955 | Endochitinase | *Hordeum vulgare* | 65.179 | 10 | 5 | 3.16*109 |
| Q88XP6 | Aspartyl/glutamyl-tRNA(Asn/Gln) amidotransferase subunit B | *Lactobacillus plantarum* | 8.921 | 5 | 5 | 4.31 * 106 |
| Q9FRV0 | Basic endochitinase | *Secale cereale* | 42.496 | 8 | 4 | 9.96*107 |
| P15326 | Alpha-amylase inhibitor/endochitinas e | *Coix lacryma* | 15.238 | 4 | 3 | 3.69*106 |

As can be seen from the information included in **Table 3**, among the enzymes included in the enzymatic extract of the present invention, enzymes such as xylanases, xylases and endochitinases are of particular interest. In addition, it can be find different enzymes that are widely known for their action in PET degradation such as aspartyl/glutamyl- tRNA amidotransferase, enzymes involved in intermediary metabolisms such as phosphoglycerate kinase, glucose 6-phosphate isomerase or beta-amylase, and enzymes involved in energy storage/obtaining processes such as ATP synthase or Glyceraldehyde-3-phosphate dehydrogenase have been also detected as present in the enzymatic extract of the invention.

In another preferred embodiment, the enzymatic extract of the invention is encapsulated in nanocapsules to stabilize them and to allow the reuse of the enzymatic extract, for example in continuous batches. Advantageously, the nanoencapsulation of the enzymatic extract of the invention allows that the depolymerization and degradation of the plastic product by the composition of the invention can be performed at room temperature. Encapsulation techniques are well known to those skilled in the art and include, for instance, nano-emulsions. In a preferred embodiment, the encapsulation of the enzymatic extract of the invention is performed in an amphoteric enzymatic matrix such as an amphoteric matrix of hydrolases, preferably hydrolases selected from: amylase-type hydrolases, Beta-amylase-type hydrolases, Gamma-amylase-type hydrolases (Glucan 1,4-alpha-glucosidase), among others. This amphoteric enzymatic matrix triggers the hydrolysis in the first reaction of the plastic depolymerization/degradation since this amphoteric matrix disrupts the hydrophobicity of the surface of the plastic product to be depolymerized/degraded, thus allowing the attack of the enzymes included in the enzymatic extract of the composition of the present invention Table 3 shows the type of enzymes that can be used as amphoteric enzymatic matrix according to the present invention.

**Table 4. Enzymes which can be used as amphoteric enzymatic matrix.**

| **Group** | **General formula/ Chemical group attacked in the plastic product** | **Enzyme** |
|---|---|---|
| Esther | 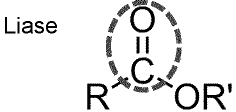 | Hydrolase |
| Acid halides | 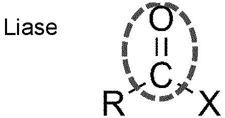 | Lipase with cofactor (CI) |
| Amide | 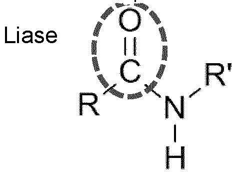 | Lipase |
| Nitrile | 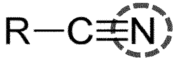 | Lipase |
| Aldehyde | 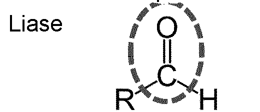 | Transferase |
| Mercaptan | 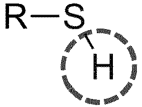 | Licase |
| Amine | 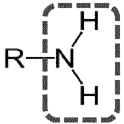 | Hydrolase |
| Alkene | 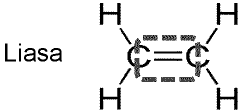 | Lipase |
| Alkyne | 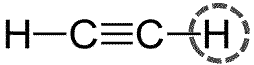 | Isomerase |
| Alkane | 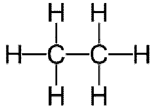 | Isomerase |

Thus, the nano-encapsulation of the enzymatic extract of the invention in an amphoteric matrix of amylase-type hydrolases of the Beta-amylase-type hydrolases, Gamma-amylase-type hydrolases (Glucan 1,4-alpha-glucosidase), promotes the initiation of hydrolysis in the first reactions of plastic degradation, mainly in the first phase of such degradation. The secondary post-hydrolysis reactions of the plastic by means of glycerol ester hydrolases catalyze the hydrolysis and also the synthesis of carboxylic ester groups. The generated mix allows, using the water solubility of the enzyme glycerol-ester hydrolases, acting on non-soluble substrates (specific ester) including water-oil interfaces. Post-esterase chain reactions, dominated by isomerases, convert the isomer of the compound into another chemical compound, basically, they are in charge of rearranging intramolecular conversions (see **Table 4**)**.**

Due to their multiple reactions resulting from the different rearrangements of atoms in the molecule, the enzymes included in the enzymatic extract of the composition of the invention are classified in EC5 category which corresponds to isomerases (https://www.enzyme-database.org/ - "ExplorEnz - The Enzyme Database"). The most important subcategory within the EC5 Isomerases category, for the enzymes included in the enzymatic extract of the present invention are the following: Hydroxy acid and derivatives thereof; Cis-trans-isomerases; intramolecular oxidoreductases; Keto-enol tautomerases; π c=c bond modifiers; and S-S disulfide bond modifiers. Depending on the type of metabolic and/or catalytic activity, they present a synonymy that is different in name, but equivalent in their chemical structures, as defined in the above types of enzymes. For clarity of the point, we include the biological/chemical nomenclature:

| **N°** | **Type** | **Catalyzed reaction** |
|---|---|---|
| 1 | Oxydoreductases | electron transfer |
| 2 | Transferases | group transfer |
| 3 | Hydrolases | hydrolysis reactions |
| 4 | Lyases | addition of groups to double bonds (or the reverse reaction) |
| 5 | Isomerases | transformation into isomeric forms |
| 6 | Ligases | formation of G-C, C-S, C-O and C-N bonds by condensation reactions (ATP needed) |

In another preferred embodiment, the concentration of the enzymatic extract in the composition is at least 50% w/v; 51% w/v, 52% w/v; 53% w/v; 54% w/v; 55% w/v; 56% w/v; 57% w/v; 58% w/v; 59% w/v; 60% w/v; 61% w/v; 62% w/v; 63% w/v; 64% w/v; 65% w/v; 66% w/v; 67% w/v; 68% w/v; 69% w/v; 70% w/v; 71% w/v; 72% w/v; 73% w/v; 74% w/v; 75% w/v; 76% w/v; 77% w/v; 78% w/v; 79% w/v; 80% w/v; 81% w/v; 82% w/v; 83% w/v; 84% w/v; 85% w/v; 86% w/v; 87% w/v; 88% w/v; 89% w/v; and 90% w/v; preferably between at least 40%p/v to 90 %p/v; more preferably between at least 50% w/v to 90% w/v. In another preferred embodiment, the concentration of the enzymatic extract in the composition is preferably between 50% w/v to 90% w/v, more preferably, between 60% w/v to 80% w/v.

In another preferred embodiment, the at least a phosphatidine is in the composition of the invention as a micro-ionized aqueous solution comprising a salt selected from the list consisting of NaCL, NaK or any combinations thereof, and wherein the concentration of phosphatidine in the composition is at least 10% w/v; 11% w/v, 12% w/v; 13% w/v; 14% w/v; 15% w/v; 16% w/v; 17% w/v; 18% w/v; 19% w/v; 20% w/v; 21% w/v; 22% w/v; 23% w/v; 24% w/v; 25% w/v; 26% w/v; 27% w/v; 28% w/v; 29% w/v; 30% w/v; 31% w/v; 32% w/v; 33% w/v; 34% w/v; 35% w/v; 36% w/v; 37% w/v; 38% w/v; 39% w/v; 40% w/v; 41% w/v; 42% w/v; 43% w/v; 44% w/v; 45% w/v; 46% w/v; 47% w/v; 48% w/v; 49% w/v; 50% w/v; 51% w/v; 52% w/v; 53% w/v; 54% w/v; 55% w/v; 56% w/v; 57% w/v; 58% w/v; 59% w/v; 60% w/v; 61% w/v; 62% w/v; 63% w/v; 64% w/v%; 65% w/v; 66% w/v; 67% w/v; 68% w/v; 69% w/v; 70% w/v; preferably between at least 10%p/v to 60 %p/v; more preferably between at least 10% w/v to 50% w/v. In another preferred embodiment, the concentration of phosphatidine in the composition is preferably between 10% w/v to 50% w/v, more preferably, between 20% w/v to 40% w/v.

As plastic waste is insoluble in water, fungal cultures producing biosurfactant plays a key role in the solubilization and/or emulsification of hydrocarbons. These mechanisms lead to desorption and, by increasing the availability of the chemical group attacked in the plastic product by the composition of the invention, ultimately enhance the biodegradation rate. Moreover, biosurfactants are an alternative to chemical surfactants because of their properties as eco-friendly, least toxic, biodegradable and exhibiting high specificity.

In a preferred embodiment, the composition of the invention is a liquid composition. Advantageously, the liquid composition of the invention is stable, i.e chemically and biologically stable. In the context of the invention, "chemically stable" refers to a composition wherein the biological entities, i.e. enzymatic extract, do not show any significant loss of activity during a defined period at room temperature, in the dark. More particularly, "chemically stable" refers to a composition wherein the loss of degrading activity of the biological entities is less than 50%, preferably less than 25%, more preferably less than 10% as compared to the degrading activity of said biological entities before introduction in the composition, during a period of time of at least 30 days, preferably at least 90 days, more preferably at least 1 year. According to the invention, the composition of the invention is chemically stable during at least 90 days at 4°C. Particularly, the loss of degrading activity of the enzymatic extract in the composition of the invention is less than 10% as compared to the degrading activity of said enzymatic extract before introduction in the composition, during a period of time of at least 90 days. In the context of the invention, the term "biologically stable" refers to a composition that does not show any subsequent bacterial, yeast of fungal proliferation during a defined period of at least 30 days, preferably at least 90 days, more preferably at least 1 year, at room temperature, in the dark.

In a second embodiment, the present invention provides the use of the composition of the present invention in the degradation of plastic products into monomers, oligomers, acids, additives, and/or other components that may be present in the plastic formulation.

According to the present invention, the plastic product comprises a polymer selected from the list consisting of PET, PTT, PBT, PElT, PLA, PLLA, PDLA, PDLLA, scPLA, PHA, P(3HB)/PHB, P(3HV)/PHV, P(3HHx), P(3HO), P(3HD), P(3HB-co-3HV)/PHBV, P(3HB-co-3HHx)/(PHBHHx), PHB5HV, PHB3HP, PHBO, PHBOd, P(3HB-co-3HV-co-4HB), PBS, PBSA, PHA, PBAT, PEF, PCL, PEA, PEN, PVC, PS, PP, PE, PU, ABS, MABS, PC, and blends/mixtures thereof.

In a preferred embodiment, the composition of the invention also acts on plastics products that might containing a polylactic acid bond to any of its forms and/or derivatives thereof, forming bonds with hydrocarbon polymers.

In another preferred embodiment, the composition of the present invention is applied to the plastic polymers in the form of a liquid composition, preferably in the form of an aqueous solution.

In a third embodiment, the present invention provides a method of degrading plastic products by the use of the composition of the present invention.

In a preferred embodiment, the method of degrading plastic of the present invention comprises a depolymerization step, performed at room temperature, wherein the plastic product is contacted with the composition of the present invention, and wherein the depolymerization step is conducted in an aqueous liquid medium.

As used herein the term "ambient temperature" or "room temperature" means a temperature between 15°C to 30°C. Such temperatures will include, 15°C, 16°C, 17°C, 8°C, 19°C, 20°C, 21 C, 22°C, 23°C, 24°C, 25°C, 26°C, 27°C, 28°C, 29°C and 30°C, particularly between 20°C to 28°C, more particularly between 23°C to 25°C.

According to the present invention, in the depolymerization step, the plastics are degraded into their respective monomers, oligomers, acids, additives, and/or other components that may be present in the plastic formulation.

In another preferred embodiment, the pH of the aqueous liquid medium wherein the depolymerization step is conducted has a pH between pH 5.0 and pH 8.0, preferably between pH 5.5-7.5.

In another preferred embodiment of the method of the present invention, the plastic product is pretreated prior to the depolymerization step, and the pretreatment step preferably includes mechanical/physical modification of the plastic product to increase the surface of contact between the plastic polymers and the composition of the present invention.

In another preferred embodiment, the pretreatment step is preferably performed by a process selected from the list consisting of cutting, crushing, grinding, milling, fractionation, desiccating, dehydration, agglomeration, granulation, or any combinations thereof.

In another preferred embodiment, the plastic polymer polymers are degraded into monomers, oligomers, acids, additives, and/or other components that may be present in the plastic formulation.

According to the present invention, the plastic polymers are selected from the list consisting of PET, PTT, PBT, PElT, PLA, PLLA, PDLA, PDLLA, scPLA, PHA, P(3HB)/PHB, P(3HV)/PHV, P(3HHx), P(3HO), P(3HD), P(3HB-co-3HV)/PHBV, P(3HB-co-3HHx)/(PHBHHx), PHB5HV, PHB3HP, PHBO, PHBOd, P(3HB-co-3HV-co-4HB), PBS, PBSA, PHA, PBAT, PEF, PCL, PEA, PEN, PVC, PS, PP, PE, PU, ABS, MABS, PC, and blends/mixtures thereof.

In another preferred embodiment, the composition of the invention also acts on plastics that might containing a polylactic acid bond to any of its forms and/or derivatives thereof, forming bonds with hydrocarbon polymers.

In another preferred embodiment, the rate of degradation of plastic products ranges from 1g /1 hour wherein the plastic is a single plastic or a blend or mixture of plastics.

### EXAMPLES

Following are examples of the invention by means of assays carried out by the inventors, which evidence the effectiveness of the product of the invention. The following examples serve to illustrate the invention and must not be considered to limit the scope thereof.

### EXAMPLE 1. Preparation procedure for the composition of the invention comprising an enzymatic extract obtained from a culture comprising at least three live Trichoderma species selected from Trichoderma harzianum, Trichoderma viridae, and Trichoderma longibratum.

The strains of *Trichoderma viridae, Trichoderma longibratum and Trichoderma harziano* were cultivated all together in a traditional bioreactor on micronized and a slightly hydrated layer of oat as solid means of cultivation and fed with glucose-maltose to obtain an aqueous solution rich in secretions but poor in Trichoderma biomass and afterward a sieving was made that allowed to pick up the spores present in this culture. The micronized oat is subjected to sterilization by controlled exposition to microwaves to avoid the degradation of the fructose contained in its germ.

These fungus spores were cultivated over an oat layer previously sterilized by means of controlled exposition to microwaves and slightly hydrated until reaching to isolate each species in a separate jar.

To avoid the annihilation of the species while living together in the culture, a transformation was realized through the exposition of the *Trichoderma longibratum* strains to radiation, transforming it by induced selection in a strain resistant to the fungicidal secretions that release the two Trichoderma species that are contained in the final product. More specifically, the original *Trichoderma longibratum* species were subjected to micro expositions to Cobalt for 50 generations, obtaining a strain with unique characteristics.

When the culture has reached the maximum growth, the fungus-containing trays are harvested and immediately diluted in the substrate, which allows the fungus to grow more slowly and the dilution of the enzymes that could affect the fungus structure during the aging of the product.

The three live Trichoderma species are cultured in the proportions: *Trichoderma harzianum* 50%, *Trichoderma viridae* 30% and *Trichoderma longibratum* (already irradiated) 20%. The culture is handled in the following levels: pH 5, temperature 17°C to 22°C, with expositions of interrupted photo periods.

The generated extract harvest implies the total collection of this by mechanical means, between days 4 and 7, its re-sowing is made from this point, using live material in all of its vegetative growth phases at 1 % or more with respect to the already hydrated nutrient (micronized sterile and hydrated oat) over culture trays.

Total protein concentration was determined according to the Bradford (1976) method. The principle of this assay is that the binding of protein molecules to Coomassie dye under acidic conditions results in a color change from brown to blue. The sample analyzed has a content of 1.1 mg/ml of protein.

Once the extract is obtained, it is lyophilized until a powder is obtained. Then, a solution is prepared with deionized water and amphoteric surfactants in a proportion of 1-5% with respect to the deionized water. As amphotheric surfactant any amphoteric surfactants, chemical and/or biological, known in the art can be used, but preferably the surfactant used is phosphatidine. A pH between 6.5 and 8 is required, preferably a pH 7. Once said solution is obtained, the powder enzymatic extract and the phosphatidine are slowly added, forming small micelles that during a process of agitation and sonification at RT temperature between 18°C to 25°C for 30 min, nano-micelles are obtained. Depending on the pH, an additional esterification step with calcium carbonate may be required.

Thus, the composition of the invention comprising the encapsulated extract into nano-micelles obtained as previously disclosed, is used for further analysis.

### EXAMPLE 2. Identification of the type of enzymes included in the enzymatic extract of the composition of the invention by electrophoresis, chromatography and proteomic analysis.

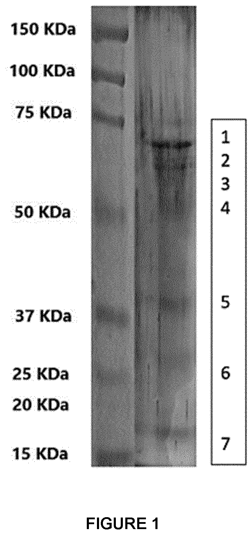

A sample of the enzymatic extract of the composition of the invention was submitted to electrophoresis in an SDS-Page gel (Western blot) for the identification of the type of enzymes included in the enzymatic extract of the composition of the invention. The results obtained by Western Blot show that the concentration of proteins was low and to identify in a better way the enzymes a silver staining in the SDS-Page gel was carried out. The results obtained shows the presence of seven different proteins based on the molecular weight in view of the control (**Figure 1**). The molecular weight of the enzymes detected is in the range of 15 kDa to 75 KDa (**Figure 1**).

Due to the limitation of the electrophoresis technique and in order to obtain a better characterization of the composition of the enzymes included in the enzymatic extract of the invention, a proteomic analysis was performed by Liquid Chromatography with tandem Mass Spectrometry (LC-MS-MS). This technique combines the separation of proteins/peptides by liquid chromatography with their subsequent analysis by mass spectrometry followed by a bioinformatics study using specialized software and protein databases such as UniProt, which allows the identification of proteins. The analysis of the samples of the enzymatic extract of the present invention was performed by the Proteomic Laboratory of the Centro Nacional de Biotecnologia (CNB-CSIC).

The result obtained by LC-MS-MS shows a similar protein pattern to that obtained by electrophoresis. **Table 5** shows the molecular weights of the peaks detected by LC-MS-MS.

**Table 5. Molecular Weight (Da) of the peaks detected by LC-MS-MS in the enzymatic extract of the invention.**

| **Peak** | **Elution Time (min)** | **Molecular Weight (Da)** |
|---|---|---|
| 1 | 28,85 | 62760 |
| 2 | 30,78 | 41127 |
| 3 | 32,27 | 29627 |
| 4 | 34,75 | 27163 |
| 5 | 36,92 | 10645 |
| 6 | 39,69 | 5785 |
| 7 | 46,5 | 1292 |
| 8 | 56,67 | 138 |

After obtaining the peptide sequences of the analyzed/detected proteins, these sequences were matched with all the species belonging to the genus Trichoderma known in the sequences databases (UniProt).

Once the results were obtained, and due to the specific absence of proteins of Trichoderma strains in the sequences databases, the protein sequences were matched with the entire UniProt database comprising plants, fungi and bacteria peptides. The results obtained are shown in **Tables 1 and 2.** No proteins with homology to any species of Trichoderma have been detected, however, proteins with homology to proteins of wheat (*Triticum aestivum*)*,* barley (*Hordeum vulgare*), oats (*Avena sativa*) and different bacteria species of the genus Lactobacillus (*L*. *brevis and L. plantarum*), have been found. Among the proteins detected and found in the enzymatic extract of the present invention, the proteins shown in **Table 3** reproduced again below, are the most important:

**Table 3. Enzymes included in the enzymatic extract of the present invention.**

| **Accession Number (Uniprot database)** | **Name** | **Specie** | **PEP Score** | **Peptides** | **Unique Peptides** | **Abundance** |
|---|---|---|---|---|---|---|
| Q8L5C6 | Xylanase inhibitor protein | *Triticum aestivum* | 60.201 | 16 | 16 | 2.08*10⁹ |
| Q03TX3 | Xylose isomerase | *Lactobacillus brevis* | 71.541 | 21 | 15 | 1.75*10⁸ |
| P11955 | Endochitinase | *Hordeum vulgare* | 65.179 | 10 | 5 | 3.16*10⁹ |
| Q88XP6 | Aspartyl/glutamyl-tRNA(Asn/Gln) amidotransferase subunit B | *Lactobacillus plantarum* | 8.921 | 5 | 5 | 4.31*10⁶ |
| Q9FRV0 | Basic endochitinase | *Secale cereale* | 42.496 | 8 | 4 | 9.96*10⁷ |
| P15326 | Alpha-amylase inhibitor/endochitinase | *Coix lacryma* | 15.238 | 4 | 3 | 3.69*10⁶ |

As can be seen from the information included in **Table 3**, among the enzymes included in the enzymatic extract of the present invention, enzymes such as xylanases, xylases and endochitinases are of particular interest. In addition, it can be find different enzymes that are widely known for their action in PET degradation such as aspartyl/glutamyl- tRNA amidotransferase, enzymes involved in intermediary metabolisms such as phosphoglycerate kinase, glucose 6-phosphate isomerase or beta-amylase, and enzymes involved in energy storage/obtaining processes such as ATP synthase or Glyceraldehyde-3-phosphate dehydrogenase have been also detected as present in the enzymatic extract of the invention.

### EXAMPLE 3. Depolymerization and biodegradation of plastic material by the composition of the invention.

Blue PET plastic fragments from a post-consumer water bottle, crushed with a particle size between 5x5 mm2 and 10x10 mm2 were put in contact with the liquid composition of the invention, in a concentration of at least 25%w/w.

Firstly, dilution of the composition of the invention comprising the enzymatic extract concentrates was performed in ultrapure water at a 1:50 ratio.

Subsequently, 2 gr of the crushed PET is mixed with 20 ml of the diluted composition of the invention. It is kept for 7 days under constant agitation and temperature, 170 rpm and 25°C, respectively, in an incubator with orbital shaking.

To monitor the depolymerizing effect by scanning electron microscopy (SEM), individual samples of the PET fragments were taken every 20 min up to a total of 80 min, and a final sample after 7 days of processing. In this last sample, a volume of 1 ml of the solution was collected for analysis by High-Performance Liquid Chromatography (HPLC) and Gas Chromatography/Mass Spectrometry (GC/MS).

Each time samples of PET fragments are extracted, the plastics are washed with ultrapure water and reserved for further analysis.

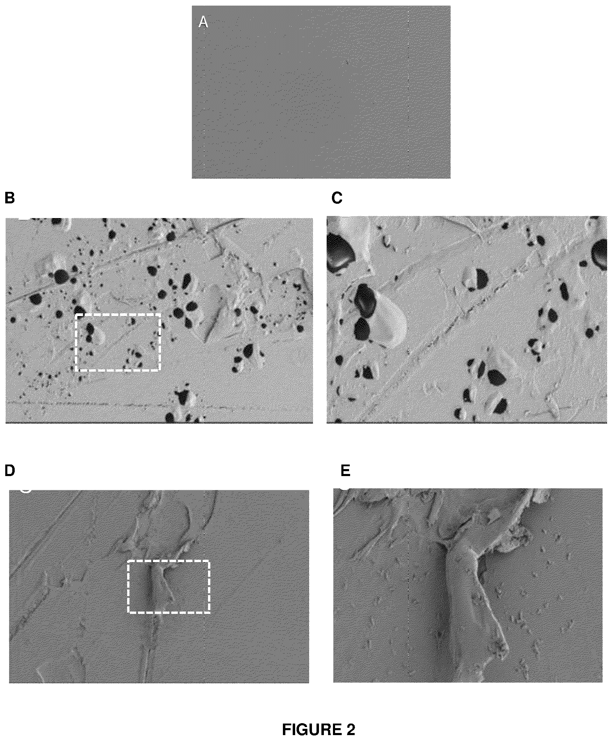

The results show that the surface of PET plastic fragments treated with the composition of the invention for 40 min (**Figures 2B and 2C**) and for 60 min (**Figures 2D and 2E**), has been completely transformed with respect to the surface of control PET plastic fragments, not treated with the composition of the invention (**Figure 2A**). Therefore, after 40 min of being in contact with the composition of the invention, the PET plastic fragment surface has been completely transformed.

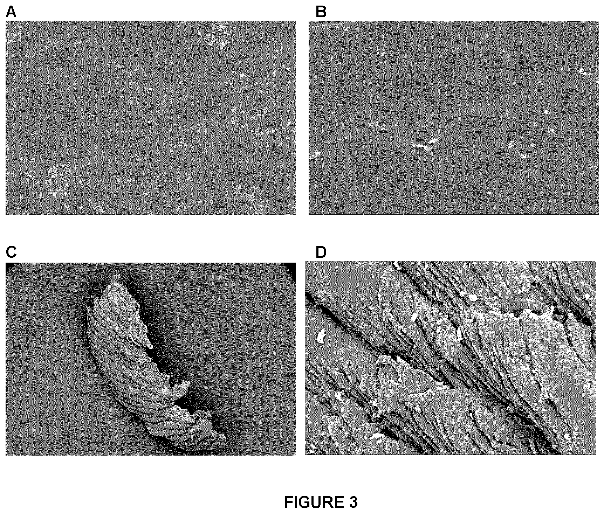

Moreover, **Figures 3A** and **3B** show SEM images (100x and 1000x, respectively) from the PET plastic fragment of a control sample, not treated with the composition of the present invention. As can be seen from these images, the PET plastic fragment-control sample shows a uniform and homogeneous surface. However, after 24 hours of being in contact with the composition of the invention, the PET plastic fragment surface has been completely transformed (**Figures 3C and 3D**).

**Figures 3B** and **3D** show in more detail how the composition of the invention operates on the PET polymer surface. While the surface of the control sample, Figure 1B, remains virtually unchanged and unmarked, the surface of the PET-treated sample,

**Figure 3D****,** shows a complete surface change. The composition of the invention acts on the polymer chains at the molecular level to degrade the plastic product.

Furthermore, an additional assay with different PET plastic fragments was also performed. The samples were mechanically crushed to reduce the size of the PET plastic fragments as above-mentioned.

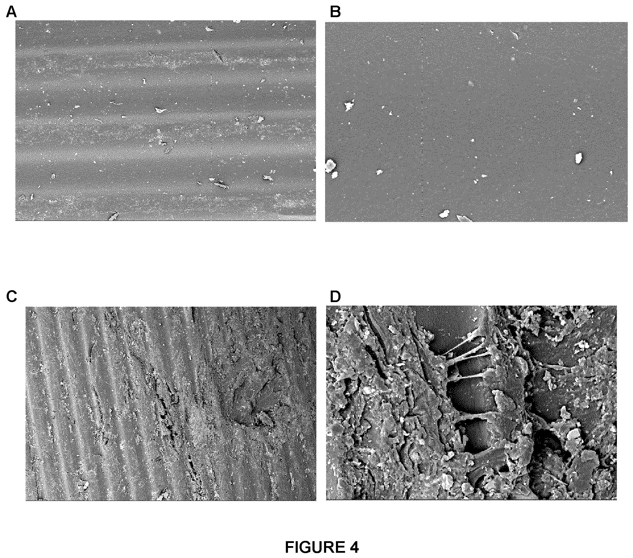

**Figure 4A** and **4B** show SEM images (100x and 1000x, respectively) from the PET plastic fragment of a control sample, not treated with the composition of the present invention. As can be seen from these images, the PET plastic fragment-control sample shows a uniform and homogeneous surface. However, after 24 hours of being in contact with the composition of the invention, the PET plastic fragment surface has been completely transformed (**Figures 4C** and **4D**).

**Figures 4B** and **4D** show in more detail how the composition of the invention operates on the PET polymer surface. While the surface of the control sample, **Figure 4B****,** remains virtually unchanged and unmarked, the surface of the PET-treated sample, **Figure 4D****,** shows a complete surface change. The composition of the invention acts on the polymer chains at the molecular level to degrade the plastic product.

Finally, an Energy-dispersive X-ray spectroscopy (EDS) analysis of the PET fragment samples after being 24h in contact with the composition of the invention revealed the presence of free bromine as a constituent of the complexes present in the original PET fragment (See **Table 6**).

**Table 6. EDS analysis of PET fragments after being 24h in contact with the composition of the invention.**

| | **Weight (%)** | | |
|---|---|---|---|
| **Element** | **Point 1** | **Point 2** | **Point 3** |
| C | 99.1 | 20.0 | 98.3 |
| Si | 0.7 | 1.3 | 1.4 |
| Br | 0.2 | 0.0 | 0.0 |
| O | 0.0 | 6.0 | 0.0 |
| Na | 0.0 | 0.1 | 0.3 |
| Al | 0.0 | 0.6 | 0.0 |
| Au | 0.0 | 72.0 | 0.0 |

The technical result herein confirms that the composition of the invention can depolymerize and degrade plastic articles at room temperature.

## Claims

1. Composition for degrading plastic products comprising an enzymatic extract obtained from a culture comprising at least three live *Trichoderma* species selected from the list consisting of Trichoderma *harzianum, viridae, polysporum, longibratum, koningii* and variants thereof, cultivated all together, obtained in laboratory and/or at industrial scale, on micronised oat as solid means of cultivation, identified as T 22, Tr 115, Tr 116, KRL -AG 2 (Rifai), including holoforms as *Hypocrea* and *Podostroma,* wherein *T. longibratum* stump was transformed by means of the exposition to radiation before being cultured with the other *Trichoderma* species, and at least a phosphatidine as a surfactant, wherein the enzymatic extract and the at least a phosphatidine are encapsulated within the composition.

2. The composition of the invention according to claim 1 wherein the encapsulation is a nano-encapsulation in an amphotheric matrix.

3. The composition of the invention according to claim 2, wherein the amphotheric matrix is a biological amphoteric matrix, preferably an amylase-type hydrolase enzimes.

4. The composition according to any one of claims 1 to 3, wherein the at least a phosphatidine is in the composition as a micro-ionized aqueous solution comprising a salt selected from the list consisting of NaCL, NaK or any combinations thereof, and wherein the concentration of phosphatidine in the composition is preferably between 10% p/v to 50%p/v.

5. The composition according to any one of claims 1 to 4, wherein the composition comprises three live *Trichoderma* species selected from stumps *Trichoderma viridae, Trichoderma longibratum* and *Trichoderma harziano,* in the proportions of 10:20:70 or 99:05:05, respectively.

6. The composition according to any one of claims 1 to 5, wherein the enzymatic extract comprises enzymes from alpha/beta hydrolase superfamily selected from the list consisting of: carboxidase-transferase-hydrolases; transferase-hydrolase-carboxidases; hydrolase-carboxidase-transferases, or any combinations thereof.

7. The composition according to any one of claims 1 to 6, wherein the plastic is selected from the list consisting of polyethylene terephthalate (PET), polyvinyl chloride (PVC), polystyrene (PS), polypropylene (PP), polyethylene (PE), polylactic acid (PLA), acrylonitrile butadiene styrene (ABS), methylmethacrylate acrylonitrile butadiene styrene (MABS), polycarbonate (PC), polyurethane (PU), nylon, and blends or mixtures thereof.

8. Use of the composition according to any one of claims 1 to 7, for degrading plastic products into monomers, oligomers, acids, additives, and/or other components that may be present in the plastic formulation.

9. Use according to claim 8, wherein the plastic is selected from the list consisting of PET, PVC, PS, PP, PE, PLA, PU, ABS, MABS, PC and blends or mixtures thereof.

10. Use according to claim 8 or 9 wherein the plastic comprises at least a polylactic acid bond to any of its forms and/or derivatives thereof, forming bonds with hydrocarbon polymers.

11. Use according to any one of claims 8 to 10, wherein the composition is applied to the plastic product in the form of a liquid composition, preferably in the form of an aqueous solution.

12. Method of degrading plastic products, wherein the method comprising a depolymerization step, performed at room temperature, preferably between 15°C and 30°C, wherein the plastic product is contacted with the composition according to any one of claims 1 to 7; and wherein the depolymerization step is conducted in an aqueous liquid medium, wherein the pH of aqueous liquid medium is preferably between pH 5.0 to 8.0.

13. The method according to claim 12, wherein the plastic product is pretreated prior to the depolymerization step, the pretreatment preferably including mechanical/physical modification of the plastic product to increase the surface of contact between the polymers and the composition, and wherein the pretreatment preferably is performed by a process selected from the list consisting of cutting, crushing, grinding, milling, fractionation, desiccating, dehydration, agglomeration, granulation, or any combinations thereof.

14. The method according to any one of claims 12 to 13, wherein the plastic is selected from the list consisting of PET, PVC, PS, PP, PE, PLA, PU, ABS, MABS, PC, and blends or mixtures thereof.

15. The method according to any one of claims 12 to 14, wherein the rate of degradation of plastic from 1g /1 hour wherein the plastic is a single plastic or a blend or mixture of plastics.
